# EUROPÄISCHE PATENTANMELDUNG

(11) **EP 3 421 018 A1**
(43) Veröffentlichungstag der Anmeldung: **02.01.2019**
(21) Anmeldenummer: 18177728.5
(22) Anmeldetag: 14.06.2018
(51) Int. Cl.: A61F 9/007, A61F 9/008, A61B 17/00, A61B 18/00

(54) **WIRKVERBINDUNG ZUM ANSCHLIESSEN EINES MEDIZINISCHEN GERÄTS**

(30) Priorität: 28.06.2017 DE 102017210875
(71) Anmelder: Geuder AG, 69126 Heidelberg (DE)
(72) Erfinder: Draheim, René, 69207 Sandhausen (DE); Pelka, Falko, 71723 Großbottwar (DE)
(74) Vertreter: Patent- und Rechtsanwälte Ullrich & Naumann

(57) **Zusammenfassung**

Eine Wirkverbindung zum Anschließen eines medizinischen Geräts über einen Versorgungspfad an eine Versorgungseinrichtung, wobei die Wirkverbindung mindestens zwei Eingriffsteile umfasst ist, dadurch gekennzeichnet, dass im verbundenen Zustand der Eingriffsteile ein Magnetfeld wirkt, wobei die Erkennung des Magnetfeldes zum Freischalten bzw. zur Aktivierung der Versorgungseinrichtung dient.

## Beschreibung

Die Erfindung betrifft eine Wirkverbindung zum Anschließen eines medizinischen Geräts über einen Versorgungspfad an eine Versorgungseinrichtung, wobei die Wirkverbindung mindestens zwei Eingriffsteile umfasst.

Aus der Praxis sind unterschiedlichste Wirkverbindungen der hier in Rede stehenden Art bekannt, beispielsweise auf dem Gebiet der Ophthalmologie. Dazu sei lediglich beispielhaft auf die DE 10 2008 056 565 A1 verwiesen, wonach es um das Einkoppeln von Licht in ein medizinisches Gerät, insbesondere in ein ophthalmologisches Handgerät, geht. Die Kopplung kann mechanisch oder magnetisch erfolgen.

Aus der Praxis ist es auch bereits bekannt, kodierte Kopplungen zu realisieren, um nämlich eine Erkennung des eigenen Zubehörs vornehmen zu können. Es gibt entsprechende Verfahren, die die RFID-Technologie nutzen, wobei es dazu erforderlich ist, dem zu erkennenden Teil einen Transponder zuzuordnen, der einen kennzeichnenden Code enthält. Zum Auslesen des Codes bzw. der Kennung ist ein besonderes Lesegerät erforderlich. Diese Technologie ist von den zu treffenden Vorkehrungen her aufwändig und daher teuer.

Der vorliegenden Erfindung liegt daher die Aufgabe zugrunde, eine Wirkverbindung zur Anwendung in der Medizintechnik, insbesondere auf dem Gebiet der Ophthalmologie, anzugeben, die die Kopplung eines ordnungsgemäßen Zubehörs feststellt. Außerdem soll sich die Wirkverbindung von wettbewerblichen Produkten unterscheiden.

Voranstehende Aufgabe ist durch die Merkmale des Anspruchs 1 gelöst. Danach ist die gattungsbildenden Wirkverbindung dadurch gekennzeichnet, dass im verbundenen Zustand der Eingriffsteile ein Magnetfeld wirkt, wobei die Erkennung des Magnetfeldes zum Freischalten bzw. zur Aktivierung der Versorgungseinrichtung dient.

Erfindungsgemäß ist eine Wirkverbindung geschaffen, die sicher stellt, dass die Versorgungseinrichtung nur dann aktiviert wird oder aktivierbar ist, wenn das "richtige" Zubehör verwendet wird. Dadurch ist ein ordnungsgemäßer Betrieb sicher gestellt, was insbesondere auf dem Gebiet der Medizintechnik zwingend erforderlich ist, jedenfalls dann, wenn es sich um Gerätschaften und Zubehör zum Zwecke chirurgischer Eingriffe, insbesondere ophthalmologischer Eingriffe, handelt. Es gilt Sicherheit zu schaffen.

Der Begriff "Eingriffsteil" ist im weitesten Sinne zu verstehen, wobei es sich hier beispielsweise um eine Steckverbindung mit Stecker und Buchse handeln kann. Regelmäßig ist die Buchse dem Gehäuse eines Geräts und ist der Stecker dem Versorgungspfad (z.B. einer Leitung) oder dem medizinischen Gerät zugeordnet. Auch ist es denkbar, dass einerseits die Versorgungseinrichtung und andererseits das medizinische Gerät eine Buchse hat, wobei der Versorgungspfad, beidseitig, jeweils mit einem Stecker ausgestattet ist. Beliebige Verbindungskombinationen und Verbindungstechniken sind denkbar.

Entsprechend den voranstehenden Ausführungen erstreckt sich das Magnetfeld zwischen dem Stecker und der Buchse im gekoppelten Zustand, so dass durch das Aufspannen und Detektion des Magnetfelds eine ordnungsgemäße Kopplung feststellbar ist.

Bei der Versorgungseinrichtung kann es sich um eine Fluidquelle oder eine Fluidsenke handeln, beispielsweise um eine Druckluftquelle. Auch kann es sich bei der Versorgungseinrichtung um Anschlüsse für Aspiration und Irrigation handeln. Entsprechend kann das medizinische Gerät ein ophthalmologisches Handgerät sein. Der Versorgungspfad wäre dann beispielsweise ein einfacher oder ein doppelter Schlauch.

Ebenso ist es denkbar, dass die Versorgungseinrichtung eine Lichtquelle ist bzw. umfasst. Dabei kann es sich beispielsweise um eine Laserlichtquelle oder um eine LED-Lichtquelle handeln. Das medizinische Gerät wäre wiederum ein ophthalmologisches Handgerät. Der Versorgungspfad wird in diesem Falle über einen Lichtleiter definiert, der auf der einen Seite mit der Versorgungseinrichtung bzw. der Lichtquelle und auf der anderen Seite mit dem Handgerät verbunden ist.

In besonders vorteilhafter Weise ist dem einen Eingriffsteil, beispielsweise der Buchse, ein Permanentmagnet zugeordnet. Dabei kann es sich im Konkreten um einen Stabmagneten handeln. Dem anderen Eingriffsteil, beispielsweise dem Stecker, könnte ein ferro- oder ferrimagnetisches Material zugeordnet sein, nämlich eine entsprechende Metalleinlage, so dass die Verbindung der Eingriffsteile bzw. die Anwesenheit des Steckers in der Buchse durch Detektion des sich zwischen dem Permanentmagneten und der Metalleinlage aufspannenden Magnetfelds feststellbar ist. Wie bereits zuvor erwähnt, ist dazu die mechanische Kopplung zwischen den Teile erforderlich.

Im Konkreten kann die Metalleinlage als Metallring oder Metallscheibe ausgeführt sein. Die Metalleinlage kann aus einem weichmagnetischen Material bestehen, beispielsweise auf MU-Metall (Permalloy).

Die Metalleinlage kann je nach dem konkret anzuschließenden Zubehör unterschiedlich dick sein und/oder aus unterschiedlichen Materialien bestehen und/oder in unterschiedlichen Positionen in dem jeweiligen Teil eingebaut sein, so dass über Unterschiede in der detektierbaren Magentfeldstärke das konkrete Zubehör erkennbar und vor allem auch unterscheidbar ist. Somit wäre nicht nur die bloße Anwesenheit des Zubehörs feststellbar, vielmehr auch unterscheidbar, um welches konkrete Zubehör es sich handelt. Entsprechend kann dann die Versorgungseinrichtung aktiviert werden, um auch insoweit eine Fehlfunktion ausschließen zu können.

Zur Detektion des Magnetfelds ist es von weiterem Vorteil, wenn ein Wirbelstromsensor verwendet wird, der in einen elektronischen Schwingkreis eingebunden sein kann. Da die konkrete Detektion des Magnetfeldes nicht Gegenstand der Erfindung ist, sei insoweit auf die diesbezügliche Literatur zur Ausgestaltung und Funktionsweise von Wirbelstromsensoren verwiesen, wie sie beispielsweise von der Firma Micro-Epsilon patentiert worden sind.

Schließlich ist es von ganz besonderem Vorteil, wenn die Vorkehrung des Permatentmagneten und der Metalleinlage weiterreichend genutzt wird, beispielsweise zur Arretierung zwischen den Eingriffsteilen, insbesondere zwischen dem Stecker und der Buchse. So lässt sich in idealer Weise eine Verbindung von Zubehör an einem Gerätesystem mittels Magnetkraft realisieren, wobei diese Verbindung gleichzeitig auch zur Erkennung des Zubehörs und der Art des Zubehörs dient.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung eines bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht den grundsätzlichen Aufbau einer erfindungsgemäßen Wirkverbindung,
- Fig. 2: in einer schematischen Ansicht das Zusammenwirken einer Buchse und eines Steckers als konkrete Wirkverbindung und
- Fig. 3: in einer schematischen Ansicht den Lichtleiterstecker und Adapter gemäß Fig. 2 im entkoppelten Zustand.

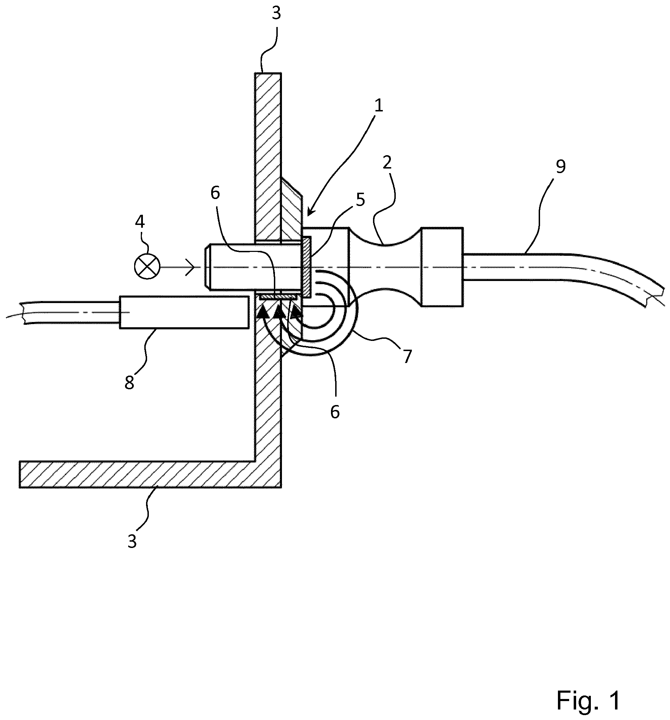

Fig. 1 zeigt in einer schematischen Ansicht eine Wirkverbindung zwischen einer Gehäusebuchse 1 und einem Lichtleitstecker 2, wobei die Gehäusebuchse 1 einem Gehäuse bzw. einer Gehäusewand 3 zugeordnet ist. Im Gehäuse bzw. hinter der Gehäusewand 3 befindet sich eine Lichtquelle 4.

Der Lichtleitstecker 2 ist mit einem Metalleinleger 5 ausgestattet, wobei es sich dabei um eine Metallplatte aus weichmagnetischem Material handeln kann.

Der Gehäusebuchse 1 ist ein Permanentmagnet 6 zugeordnet. Ist der Lichtleitstecker 2 ordnungsgemäß in die Gehäusebuchse 1 eingesteckt, erstreckt sich zwischen dem Metalleinleger 5 und dem Permanentmagneten 6 ein Magnetfeld 7, welches über einen Sensor 8 detektierbar ist. Aufgrund der konkreten Ausgestaltung und Anordnung des Metalleinlegers 5 und des Permanentmagneten 6 lässt sich nicht nur die bloße Anwesenheit des Lichtleitsteckers 2 detektieren, vielmehr auch das konkrete Zubehör, nämlich bei dem hier gewählten Ausführungsbeispiel die konkrete Lichtleitfaser 8, die über den Lichtleitstecker 2 mit der Lichtquelle 4 verbunden wird, nämlich zum Zwecke der Einkopplung von Licht in ein in Fig. 1 nicht gezeigtes Handgerät.

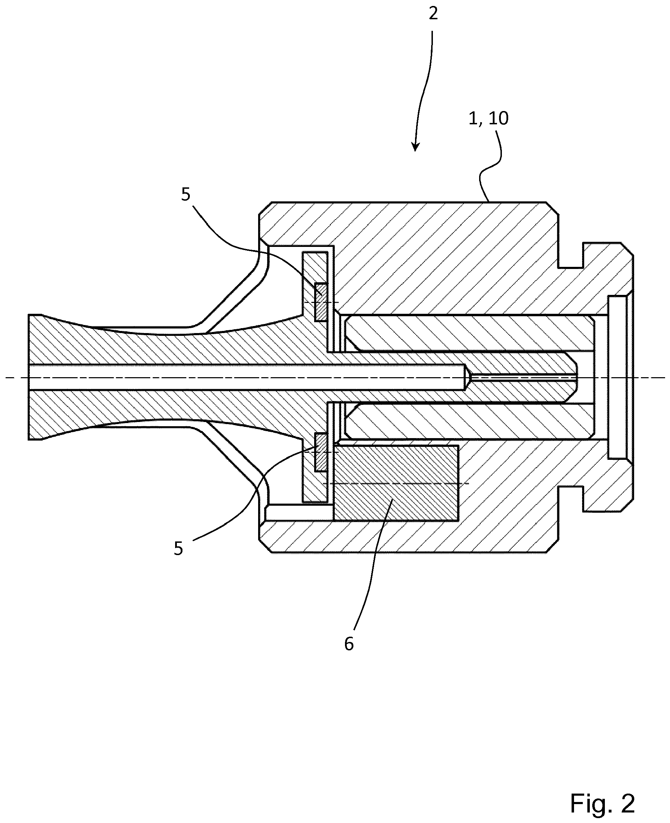

Fig. 2 zeigt in einer geschnitten Seitenansicht ein Ausführungsbeispiel der Anordnung eines Lichtleitsteckers 2 und eines Adapters 10, der als Teil der Gerätebuchse 1 zu verstehen ist.

Fig. 2 zeigt außerdem den der Gerätebuchse 1 zugeordneten Stabmagneten 6, der im zusammengesteckten Zustand gemäß Fig. 2 eine definierte Anordnung zu einem Metalleinleger 5 hat, der bei dem im Fig. 2 gewählten Ausführungsbeispiel als Metallring ausgeführt ist. Aus der Anordnung von Metallring 5 und Stabmagnet 6 ergibt sich ein Magnetfeld, welches über einen in Fig. 2 nicht gezeigten Detektor detektierbar ist.

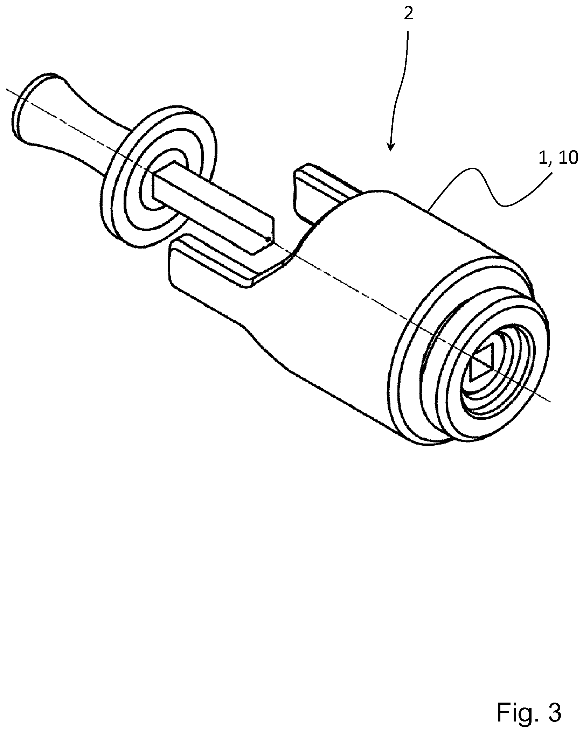

Fig. 3 zeigt ein Gegenstand aus Fig. 2 im nicht zusammengesteckten Zustand, wonach nämlich der eigentliche Stecker 2 aus der Gerätebuchse 1 herausgezogen ist.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Gehäusebuchse, Gerätebuchse
- 2: Lichtleitstecker
- 3: Gehäusewand
- 4: Lichtquelle
- 5: Metalleinlage, Metallscheibe, Metallring
- 6: Permanentmagnet, Stabmagnet
- 7: Magnetfeld
- 8: Sensor, Wirbelstromsensor
- 9: Lichtleitfaser
- 10: Adapter

## Patentansprüche

1. Wirkverbindung zum Anschließen eines medizinischen Geräts über einen Versorgungspfad an eine Versorgungseinrichtung, wobei die Wirkverbindung mindestens zwei Eingriffsteile umfasst,
**dadurch gekennzeichnet, dass** im verbundenen Zustand der Eingriffsteile ein Magnetfeld wirkt, wobei die Erkennung des Magnetfeldes zum Freischalten bzw. zur Aktivierung der Versorgungseinrichtung dient.

2. Wirkverbindung nach Anspruch 1, **dadurch gekennzeichnet, dass** die Eingriffsteile als Stecker und Buchse ausgeführt sind, wobei sich das Magnetfeld zwischen dem Stecker und der Buchse im gekoppelten Zustand erstreckt.

3. Wirkverbindung nach Anspruch 1 oder 2, **dadurch gekennzeichnet, dass** die Versorgungseinrichtung eine Fluidquelle oder eine Fluidsenke, beispielsweise eine Druckluftquelle, und das medizinische Gerät ein ophthalmologisches Handgerät und der Versorgungspfad ein Schlauch ist.

4. Wirkverbindung nach Anspruch 1 oder 2, **dadurch gekennzeichnet, dass** die Versorgungseinrichtung eine Lichtquelle, vorzugsweise eine Laser- oder LED-Lichtquelle, das medizinische Gerät ein ophthalmologisches Handgerät und der Versorgungspfad ein Lichtleiter ist.

5. Wirkverbindung nach einem der Ansprüche 1 bis 4, **dadurch gekennzeichnet, dass** dem einen Eingriffsteil, beispielsweise der Buchse, ein Permanentmagnet, vorzugsweise ein Stabmagnet und dem anderen Eingriffsteil, beispielsweise dem Stecker, eine ferro- oder ferrimagnetische Metalleinlage zugeordnet ist, so dass die Verbindung der Eingriffsteile bzw. die Anwesenheit des Steckers in der Buchse durch Detektion des sich zwischen dem Permanentmagneten und der Metalleinlage aufspannenden Magnetfels feststellbar ist.

6. Wirkverbindung nach Anspruch 5, **dadurch gekennzeichnet, dass** die Metalleinlage als Metallring oder als Metallscheibe ausgeführt ist.

7. Wirkverbindung nach Anspruch 6, **dadurch gekennzeichnet, dass** die Metalleinlage aus einem weichmagnetischen Material besteht, beispielsweise aus MU-Metall (Permalloy).

8. Wirkverbindung nach einem der Ansprüche 5 bis 7, **dadurch gekennzeichnet, dass** die Metalleinlage je nach dem konkret anzuschließenden Zubehör unterschiedlich dick sein kann und/oder aus unterschiedlichen Materialien bestehen kann und/oder in unterschiedlichen Positionen eingebaut sein kann, so dass über Unterschiede in der detektierbaren Magnetfeldstärke das konkrete Zubehör erkennbar und unterscheidbar ist.

9. Wirkverbindung nach einem der Ansprüche 1 bis 8, **dadurch gekennzeichnet, dass** zur Detektion des Magnetfelds ein Wirbelstromsensor verwendet wird.

10. Wirkverbindung nach einem der Ansprüche 1 bis 9, **dadurch gekennzeichnet, dass** das Zusammenspiel des Permanetmagneten und der Metalleinlage zur magnetischen Arretierung zwischen den Eingriffsteilen, insbesondere von Stecker und Buchse, dient.
